# EUROPEAN PATENT APPLICATION

(11) **EP 2 253 221 A1**
(43) Date of publication of application: **24.11.2010**
(21) Application number: 10466012.1
(22) Date of filing: 18.05.2010
(51) Int. Cl.: A23C 9/137, A23L 1/015, A23L 1/052, A23L 1/064, A23L 1/28, A23L 1/308, A23L 1/314, A61K 36/06, A61P 3/04

(54) **Semi-finished product of edible mushrooms, method of its production and use**

(30) Priority: 22.05.2009 CZ 20090325
(71) Applicant: Polach, Tomas, 739 21 Paskov (CZ)
(72) Inventor: Polach, Tomas, 739 21 Paskov (CZ)
(74) Representative: Holasova, Hana

(57) **Abstract**

Semi-finished product of edible mushrooms, mainly industrially grown, contains fibre in dearomatised edible mushrooms without typical taste and odour, with reduced water contents in dry matter. The semi-finished product of edible mushrooms is produced in such a way that the mushrooms are boiled at least once in hot water. Then the broth is poured off, the mushrooms are rinsed and then wrung and they can be used for various food products.

## Description

### Technical Field

The invention relates to a semi-finished product of edible mushrooms, mainly industrially grown wood-destroying fungi, the method of its production and use in food products.

### Contemporary State of the Art

At the present time there increases the number of people suffering from overweight and there also increases the number of people with digestive tract cancer, heart diseases and other different civilization diseases.

It is known on long-term basis that products with fibre contents can be used for weight reduction as well as prevention of various civilisation diseases.

In this respect, there are known the beneficial characteristics of mushrooms for healthy nutrition as they contain as much as 30% of fibre in mass, together with other health-beneficial substances.

The current use of mushrooms - whether industrially grown or nature-harvested is mostly limited to mushroom dishes or as a means for flavouring of meat dishes. Typical taste and aroma features of mushrooms prevent further expansion in food processing industry.

### Nature of the Invention

The aim of the invention use the edible mushrooms, mainly the industrially grown wood-destroying fungi with higher fibre contents compared to many other mushrooms and with stronger structure, for production of a semi-finished product usable as an ingredient or basic item of the most variable range of food products. This can be reached according to the invention by a semi-product of edible mushrooms based on the fact that it contains fibre of de-aromatised water-boiled mushrooms without typical taste and odour, with water contents reduced below 60 % by weight.

For long-term storage and further processing it is favourable for the water contents in dry matter to be max. 12% vol.

A simple way of obtaining the semi-product from edible mushrooms according to the invention is based on the fact that edible mushrooms are boiled at least once in hot water. Then the broth is poured off, the edible mushrooms are rinsed and wrung. For complete de-aromatisation it is purposeful for the edible mushrooms to be re-boiled in hot water once more, then the broth is poured off, the edible mushrooms are rinsed and wrung again. One rinsing is sufficient for meat products, it is suitable to repeat the process of de-aromatisation for pastry products. The necessity of repeated boiling is affected by the method of mushrooms growing - the industrially grown mushrooms are less treatment intensive and the natural material is much more aromatic and it requires repetition of the rinsing process. The rinsing is followed by wringing, drying and the method is selected depending on the preservation and conservation needs. De-watering or drying is important for use in case of food products where the natural water contents is completed with liquid (broth, syrup) with the taste of basic ingredient completed or replaced by the fibre from the volume point of view.

In case that the semi-product of edible mushrooms is consequently used, the water contents in dry matter is consequently lowered below 60% by weight. But in case of long-term storage it is purposeful to dry the semi-product so as to reach water contents below 12% of weight in dry matter.

It seems that the semi-product should be suitably used in meat products, ion which the semi-product can be used proportionally to meat up to the ratio of 1:1.

Similarly, the semi-product can be used in pastry products, milk products and as an additive to drinks.

### Sample Version of the Invention

The semi-product according to this invention consists of de-aromatised edible mushrooms with reduced water contents in dry matter below 60% by weight. Preferentially, there are considered the industrially grown edible mushrooms, of which wood-destroying fungi seem to be favourable from the point of view of fibre - mainly oyster fungi. The semi-product of de-aromatised mushrooms has no taste and smell. For storage purposes, water contents in dry matter reduced to 12% by weight is recommended.

The method of the semi-product production is based on the fact that the edible mushrooms are boiled at least once in hot water, then the broth is poured off and the edible mushrooms are rinsed and then wrung so as to reach water contents below 60% by weight. In case of residual aroma to stay in mushrooms, the mushrooms are boiled once again in hot water, after pouring off the broth they are re-rinsed and then wrung so as to reach water contents below 60% by weight. But for example in case of lemon fungi one boiling process of 20 minutes was sufficient, while oyster fungi had to be boiled twice for approximately 20 minutes in hot water.

After reaching the water contents of 60% by weight, the semi-product consisting of de-aromatised edible mushrooms is ready for consequent use. Depending on the way of use, the de-aromatised edible mushrooms are cut to large pieces of approximate size of 20-50 mm, they are crushed to pieces of 10 - 20 mm, they are crushed to fine crush of 5 mm pieces or to a paste of mixed or milled mushrooms.

In all of the above stated size, the edible mushrooms after de-aromatisation can be dried to water contents below 12% of weight for the purpose of long-time storage.

### Examples of use in meat products

### Example No. 1

In hamburgers, minced meat, there was used 0,5 kg of rough crushed semi-product of de-aromatised mushrooms per 1 kg of meat - the de-aromatised fibre de-watered to 60% by weight, completed with 0,5 l of water or meat broth, which is better. After mixing and maturing, there developed a mix of meat and de-aromatised fibre that was processed in a usual way in compliance with a concrete recipe after 1-2 hours.

### Example No. 2

In collared pork, white pudding and pates, one half of meat was replaced with the semi-product of de-aromatised edible mushrooms - in the form of rough pieces in collared pork, in the form of fine crushed pieces in case of white pudding and in the form of pasta in case of pate. 1 kg of the semi-product was completed with 1 l of high-quality broth of pork kin. Such a prepared mixture was further processed according to the appropriate recipe after expiration of approximately 2 hours.

### Example No. 3

In case of preparing goulash, ragout, etc. the meat was completed - in 1:1 ratio - with the preparation of de-aromatised mushrooms. Then, usual recipe was applied for preparation of goulash and ragout.

In all the recipes, there was used the 1:1 ratio of semi-product and the meat part, when the meat product kept its original taste, there was significantly reduced the nutrition value of the foods significantly enriched with fibre. The fibre volume reached the recommended daily value within usual dose of the food. It is apparent that even smaller portion of the semi-product can be used in meat products.

The use is not limited to meat products. The semi-product of de-aromatised edible mushrooms can be used in pastry products in the form of a semi-product for fillings and toppings or new pastry products can be made on its base as you can see here below.

### Example No. 4

Plum jam desert was made of 0,5 kg of rough de-aromatised fibre de-watered to 50% by weight and 0,3 kg of plum jam, 0,5 l of rum, 0,3 l of water and + aroma. While being warm, the mixture was completed with 0,2 l of water with pastry gelatine as the bonding agent and it was shaped. After cooling down, the desert was ready for use. The desert has a very low caloric value and high fibre contents.

### Example No. 5

Lemon desert was made of 0,5 kg of fine de-aromatised fibre de-watered to 50% by weight, one lemon and 4 spoons of bee honey (approximately 0,1 kg) . The lemon was mixed with honey under warm conditions, while adding 0,2 l of water with pastry gelatine per 0,5 kg of semi-product. Then it was shaped and cooled down. Again, this is a desert with low caloric value and high fibre contents.

The semi-product can also be used in milk products, as it can be seen on the basis of the below stated sample.

### Example No. 6

0,2 kg of fine crushed de-aromatised edible mushrooms was mixed with 0,5 kg of strawberries, flavoured with lemon juice and sweetened with honey. The mixture was boiled in such a way so as to preserve the fruit pieces. After cooling the boiled mixture down, it was added to yoghurt.

Apparently, the semi-product can be combined with any fruits or vegetables. The semi-product is suitable as a part of drinks, mainly cocktails, as it can be seen in the following example.

### Example No. 7

Mix 1 lemon without lemon stones and 50 g of bee honey for sweetening with 0,05 of the semi-product of de-aromatised edible mushrooms in the form of dried powder and 1 l of water. After liquidising the mixture, you will get an absolutely dietary cocktail rich in volume. A very tasty cocktail can be obtained in case of using strawberries instead of lemon and by using milk instead of water.

### Industrial Applicability

The invention is applicable in food processing industry for reduction of caloric values of food and for an increase of fibre contents and contents of other health-favourable substances in food products.

## Claims

1. Semi-product of edible mushrooms, mainly industrially grown, **characterised by** the fact that it contains fibre of de-aromatised water-boiled mushrooms without typical taste and odour, with water contents reduced below 60 % by weight.

2. Semi-product according to claim 1, **characterised by** the fact that water contents in dry matter is max. 12% by weight for long-term storage.

3. Method of production of the semi-product according to claims 1 and 2 **characterised by** the fact that the mushrooms are boiled at least once in hot water, after pouring off the broth they are rinsed and then wrung.

4. Method of production according to claims 1 up to 3 **characterised by** the fact that after at least one more boiling, the edible mushrooms are poured off the broth, re-rinsed and wrung.

5. Method of production according to claims 1 up to 4 **characterised by** the fact that after the last wringing the mushrooms are dried to water contents below 12% by weight in dry matter.

6. Semi-product according to any of the claims 1 up to 5 **characterised by** the fact that it is used in meat products in ratio to meat up to 1:1.

7. Semi-product according to any of the claims 1 up to 5 **characterised by** the fact that it is used for pastry products.

8. Semi-product according to any of the claims 1 up to 5 **characterised by** the fact that it is used in quantity of up to 12% by volume in drinks.

9. Semi-product according to any of the claims 1 up to 5 **characterised by** the fact that after flavouring and addition of bonding agent and natural sweetener it is used as an ingredient for yoghurts.
